# EUROPEAN PATENT APPLICATION

(11) **EP 4 631 517 A1**
(43) Date of publication of application: **15.10.2025**
(21) Application number: 24020111.1
(22) Date of filing: 08.04.2024
(51) Int. Cl.: A61K 38/03, A61K 38/07, A61P 17/02

(54) **SKIN WOUND TREATMENT**

(71) Applicant: The Boots Company plc, Nottingham NG90 1BS (GB)
(72) Inventor: Bell, Michael David, Alfreton Derbyshire, DE55 6EL (GB); Bradley, Eleanor Jane, Beeston Nottingham, NG9 2ES (GB); Sherratt, Michael John, Whaley Bridge High Peak, SK23 7SB (GB); El Houni, Zeyad, Eccles Manchester, M30 0ZB (GB); Ozols, Mattiss, Loughton, IG10 3LL (GB); Eckersley, Alexander, Deansgate Manchester, M3 4JN (GB)
(74) Representative: Mukhtar, Amira

(57) **Abstract**

According to the present invention, there is provided a pharmaceutical composition for use in a method of treating a wound in the skin of a subject and methods of using the pharmaceutical composition, the pharmaceutical composition comprising a tetrapeptide having the amino acid sequence U-XXGD-Z wherein G is used to denote amino acid Glycine and D is used to denote amino acid Aspartic acid, as per the internationally recognised single letter code for amino acids. X denotes an amino acid selected from the group consisting of Glutamic acid (E), Lysine (K), Leucine (L), Alanine (A), Isoleucine (I), Arginine (R) and mixtures thereof. At the N-terminal end, U is selected from the group consisting of H, -CO-R¹, - SO₂-R¹ or a biotinyl group. At the C-terminal end, Z is selected from the group consisting of OH, O R¹, NHR¹ or NR¹R². R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N.

## Description

### Technical Field

The present invention relates to pharmaceutical compositions for use in the treatment of topical skin wounds in a subject, and methods of treatment of skin wounds.

### Background to the invention

A wound is created after injury to the skin. This wound then transforms into a scar which can fade over time, or can be permanent. The wound repair process can be divided into: a) coagulation and inflammation which occurs after hours and days; b) the formation of granulation tissue which requires re-epithelialisation (days 3 - 10), angiogenesis (days 4 - 21) and fibroplasia (days 3 - 21) and c) scar formation (day 21 to 1 year) which requires extracellular matrix (ECM) remodelling, wound contraction and apoptosis.

To heal a wound effectively, the skin barrier needs to be restored quickly to prevent further damage and infection (healing speed). After closing, wound remodelling occurs to reduce the severity of or prevent scarring (healing maturity).

The National Health Service in the United Kingdom currently spends in excess of $8billion on wound care, with a predominant focus on wound care in the elderly. Despite this, there are no evidence based effective topical solutions that improve the healing of either relatively superficial or deeper skin wounds. In addition, existing wound treatments (for example topical products from brands such as Savlon^{®}, TCP^{®}; and dressings such as silicone or silver impregnated dressings) are not compatible with all skin and wound types.

There is therefore a need for novel wound treatments and compositions for use in wound treatment that improve the healing of skin wounds and that are suitable for all skin and wound types.

WO2022106056A1 discloses peptides for use in cosmetic beauty applications, and compositions comprising said peptide. The document teaches the benefits of the peptide in relation to enhancing production of dermal ECM proteins (such as fibrillin, fibronectin, decorin and collagen) in aging skin.

### Summary of the invention

The present inventors identified that specific peptides are effective in accelerating wound closure (healing speed) thus enhancing the quality of the repair (healing maturity), and providing an improved outcome for patients and consumers. They have also identified that, as the peptides are drawn from peptides naturally occurring within the skin, the peptides have the potential to be effective for all skin types, ages and genders.

The peptides described herein support remodelling of the ECM and activation of fibroblasts and so encourage the formation of granulation tissue and scar formation, thus hastening and enhancing the repair of wounded skin.

According to the present invention, there is provided in a first aspect a pharmaceutical composition for use in a method of treating a wound in the skin of a subject, the pharmaceutical composition comprising a tetrapeptide having the amino acid sequence U-XXGD-Z wherein G is used to denote amino acid Glycine and D is used to denote amino acid Aspartic acid, as per the internationally recognised single letter code for amino acids. X denotes an amino acid selected from the group consisting of Glutamic acid (E), Lysine (K), Leucine (L), Alanine (A), Isoleucine (I), Arginine (R) and mixtures thereof. At the N-terminalend, U is selected from the group consisting of H, -CO-R¹, -SO₂-R¹ or a biotinyl group. At the C-terminal end, Z is selected from the group consisting of OH, O R¹, NHR¹ or NR¹R²; R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N.

In a second aspect, the present invention provides a method of treating a wound in the skin of a subject, wherein the method comprises administering to the wounded skin of a subject a pharmaceutical composition comprising a tetrapeptide having the amino acid sequence U-XXGD-Z wherein G is used to denote amino acid Glycine and D is used to denote amino acid Aspartic acid, as per the internationally recognised single letter code for amino acids. X denotes an amino acid selected from the group consisting of Glutamic acid (E), Lysine (K), Leucine (L), Alanine (A), Isoleucine (I), Arginine (R) and mixtures thereof. At the N-terminal end, U is selected from the group consisting of H, -CO-R¹, -SO₂-R¹ or a biotinyl group. At the C-terminal end, Z is selected from the group consisting of OH, O R¹, NHR¹ or NR¹R²; R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N.

It is surprising and beneficial that certain specific peptides as defined herein are noticeably effective in accelerating wound closure (healing speed) thus enhancing the quality of the repair (healing maturity). It is further advantageous that these specific identified combinations have the potential to be effective for all skin types, ages and genders. The claimed invention therefore presents an unexpected and highly beneficial new treatment regimen for wound healing, thus providing an improved outcome for patients and consumers.

### Detailed description of the invention

The present invention is directed toward a pharmaceutical composition comprising a tetrapeptide for use in in a method of treating a wound in the skin of a subject, and method of treating a wound in the skin of a subject. In some embodiments, the peptide is a tetrapeptide.

The pharmaceutical composition and method enable the production of proteins of the dermal extracellular matrix (ECM), including at least fibrillin, fibronectin, decorin and collagen.

The method comprises administering to the wounded skin of said subject the pharmaceutical composition (e.g. in a therapeutically effective amount).

The subject may be a mammal, such as a human, primate, dog, cat, cattle, pig, horse or sheep. In preferred embodiments, the subject is a human.

### Tetrapeptides

The present invention is directed toward a pharmaceutical composition comprising a tetrapeptide for use in the treatment of a skin wound in a subject, or a method comprising administering to the wounded skin of a subject a pharmaceutical composition comprising a first tetrapeptide.

The tetrapeptide has the generic formulation U-XXGD-Z wherein G is used to denote amino acid Glycine and D is used to denote amino acid Aspartic acid, as per the internationally recognised single letter code for amino acids. X denotes an amino acid selected from the group consisting of Glutamic acid (E), Lysine (K), Leucine (L), Alanine (A), Isoleucine (I), Arginine (R) and mixtures thereof. At the N-terminal end, U is selected from the group consisting of H, -CO-R¹, -SO₂-R¹ or a biotinyl group. At the C-terminal end, Z is selected from the group consisting of OH, OR¹, NHR¹ or NR¹R². R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N.

The tetrapeptide of the present invention is preferably selected from the group consisting of SEQ ID No: 1, SEQ ID No: 2, SEQ ID No: 3, SEQ ID No: 4, SEQ ID No: 5, SEQID No: 6, SEQ ID No: 7, SEQID No: 8, SEQ ID No: 9, SEQ ID No: 10, SEQ ID No: 11, SEQ ID No: 12, SEQ ID No: 13, SEQ ID No: 14, SEQ ID No: 15, SEQ ID No: 16, SEQ ID No: 17, SEQ ID No: 18, SEQ ID No: 19, SEQ ID No: 20, SEQ ID No: 21, SEQ ID No: 22, SEQ ID No: 23, SEQ ID No: 24, SEQ ID No: 25, SEQ ID No: 26, SEQ ID No: 27, SEQ ID No: 28, SEQ ID No: 29 and SEQ ID No: 30.

In another embodiment of the present invention the tetrapeptide is preferably selected from the group consisting of SEQ ID No: 1, SEQ ID No: 12, SEQ ID No: 20 and SEQ ID No: 27.

In another embodiment of the present invention the tetrapeptide is preferably selected from the group consisting of SEQ ID No: 1.

In another embodiment of the present invention the tetrapeptide is preferably selected from the group consisting of SEQ ID No: 12.

In another embodiment of the present invention the tetrapeptide is preferably selected from the group consisting of SEQ ID No: 20.

In another embodiment of the present invention the tetrapeptide is preferably selected from the group consisting of SEQ ID No: 27.

In a preferred embodiment the tetrapeptides of the present invention is selected from the group consisting of U-EKGD-Z, U-ELGD-Z, U-EAGD-Z, U-EIGD-Z, U-ERGD-Z, U-KEGD-Z, U-KLGD-Z, U-KAGD-Z U-KIGD-Z, U-KRGD-Z, U-LEGD-Z, U-LKGD-Z, U-LAGD-Z, U-LIGD-Z, U-LRGD-Z, U-IEGD-Z, U-IKGD-Z, U-ILGD-Z, U-IAGD-Z, U-IRGD-Z, U-REGD-Z, U-RKGD-Z, U-RLGD-Z, U-RAGD-Z, U-RIGD-Z, U-AEGD-Z, U-AKGD-Z, U-ALGD-Z, U-AIGD-Z and U-ARGD-Z. In another embodiment, the tetrapeptides is selected from the group consisting of U-EKGD-Z, U-LKGD-Z, U-IRGD-Z and U-AKGD-Z. In another embodiment the tetrapeptide is U-EKGD-Z. In another embodiment the tetrapeptide is U-LKGD-Z. In another embodiment the tetrapeptide is U-IRGD-Z. In another embodiment the tetrapeptide is U-AKGD-Z.

Where, at the N-terminal, U is H then the amino acid is not modified. When, at the C-terminal, Z is OH then the amino acid is not modified. The tetrapeptide is thus not in derivatised form. When other than U is H and Z is OH, then the tetrapeptide is derivatised. Derivation of the tetrapeptide is intended to increase the bioavailability of the peptide by improving the ability of the tetrapeptide to pass through the skin. An increase in bioavailability can also be achieved through vectoring, for example by encapsulation of the peptide.

In a preferred embodiment of the present invention the tetrapeptide is modified at the N-terminal and/or the C-terminal end.

In a preferred embodiment of the present invention, R¹ and/or R² is an alkyl chain of from 1 to 24 carbon atoms, preferably a lipophilic alkyl chain of 3 to 24 carbon atoms.

In a further preferred embodiment of the present invention U is an acyl group -CO-R¹ and Z is selected from the group consisting of OH, methoxy, ethoxy and NH₂. Z is preferably OH In a further embodiment, U is preferably independently selected from the group consisting of octanoyl (C8), decanoyl (C10), lauroyl (C12), myristoyl (C14), palmitoyl (C16), stearoyl (C18), biotinoyl, elaidoyl, oleoyle and lipoyle. In a preferred embodiment of the present U is selected from lauroyl (C12), myristoyl (C14) and palmitoyl (C16).

In a further preferred embodiment Z is OH and U is independently selected from the group consisting of palmitoyl (C16), myristoyl (C14) and lauroyl (C12). Most preferably U is palmitoyl (C16) and Z is OH.

The tetrapeptides may comprise amino acids in the D- or L- configuration. The tetrapeptides may comprise an acid C-terminus such as -COzH. Alternatively, the tetrapeptide may comprise an amide C-terminus such as -CONH₂, -CONHR or CONR₂, wherein R is an alkyl chain of preferably from 1 to 24 carbon atoms.

The amino acids making up the tetrapeptides according to the invention may be optically pure, be made up of L or D isomers or a mixture of them. L isomers are those present in the natural state and may be preferred.

The present invention also envisages further derivatives of the tetrapeptide, including for example modification and/or addition of a chemically functional group to one or more of the amino acids but without a change in the carbon skeletal. The present invention also envisages further analogues of the tetrapeptide, including modification and/or addition of a chemically functional group to one or more of the amino acids with a change in the carbon skeletal and complexes of the tetrapeptide with other species such as a metal ion (e.g. copper, zinc, manganese, magnesium, and others).

Tetrapeptides may be found in the form of salts, including hydrochloric salt, or acetate.

### Pharmaceutical Composition

The pharmaceutical composition defined in the present invention is a composition for application to a wound or any other region where skin healing is desired. The tetrapeptide is preferably incorporated into the pharmaceutical composition in amount of from 0. 10ppm to 10,000ppm, preferably from 0.50ppm to 5,000ppm, more preferably from 1ppm to 1000ppm, and most preferably from 1ppm to 500ppm These are based on a % w/w basis. Thus 100,000ppm is 10% by weight of the composition.

The pharmaceutical composition may be aqueous or non-aqueous and comprise of a single-phase system or multiple phase system. The composition may include but is not limited to liquids, gels, balms, oils or solids. For example, the composition may be an emulsion, cream, gel, serum, spray, or oil. The composition may be impregnated within an article such as a dressing or patch, and applied to a wound. For example, the dressing or patch may be gauze, lint, cotton wool, cloth, semi-permeable film (e.g. polyurethane film), semi-permeable foam (e.g. silicone), hydrogel (e.g. containing methacrylate and/or polyvinyl pyrrolidine), hydrocolloid (e.g. containing carboxymethylcellulose, gelatin and/or pectin), hydrofiber (e.g. containing sodium carboxymethyl cellulose) or alginate (e.g. containing sodium and/or calcium alginate). Single or multiple phase compositions are envisaged. Multiphase systems include but are not limited to microemulsions, emulsions, and products with discrete separate phases. Emulsions include water-in-oil, oil-in-water emulsions and multiple emulsions (water in oil in water or oil in water in oil for example). Products with discrete separate phases include bi or triphasic systems where the individual water or oil phases can be visibly seen.

Where the composition is aqueous, it preferably comprises from 10% to 99.9% by weight water. In a preferred embodiment, aqueous compositions comprise from 20% to 80 % by weight water. In a preferred embodiment, aqueous compositions comprise from 40% to 70% by weight water. Where the composition is non-aqueous it preferably comprises 0% to up to 10% water, more particularly from 0.1 to 8%, most preferably from 0.5 to 5% water.

Where the composition is an emulsion it comprises an oil and a water phase. The oil phase of an emulsion can be provided by any suitable oily component. Suitable oils for the oil phase may comprise for example: a) hydrocarbon oils, such as paraffin or mineral oils; b) waxes, such as beeswax or paraffin wax; c) natural oils, such as sunflower oil, apricot kernel oil, shea butter or jojoba oil; d) silicone oils, such as dimethicone, silicone elastomer, cyclomethicone or cetylidimethicone; e) fatty acid esters and ethers, such as isopropyl palmitate or isopropyl myristate and polypropylene glycol-15 stearyl ether; f) fatty alcohols, such as cetyl alcohol or stearyl alcohol; or g) mixtures thereof, for example, the blend of waxes available commercially under the trade name Cutina (BASF).

The emulsion may comprise 0.1% to 55% by weight of the emulsion of oil phase. In one embodiment, the emulsion may comprise 3% to 25% by weight of the emulsion of oil phase, more preferably from 5% to 20% by weight of the emulsion of oil phase. In an alternative embodiment, the emulsion may comprise 10% to 50% by weight of the emulsion of oil phase, more preferably from 25 to 50% by weight of the emulsion of oil phase.

Preferably the oil phase of the emulsion comprises oil at a level between 50% and 100% by weight of the oil phase. More preferably the oil phase comprises oil at a level of from 60% to 100%, more preferably from 70% to 100%, and even more preferably from 80% to 100% by weight of the oil phase. Alternatively, the oil phase of the emulsion may comprise a combination of oil, wax or butter. Waxes and butters are hydrocarbons that consist of long aliphatic alkyl chains and may include aromatic groups. They are generally lipophilic and typically solid or malleable at room temperature. Melting points vary depending on the alkyl chain, chain length and associations. Silicone waxes are preferred type of suitable wax based on alkylmethylsiloxane. Oils are typically lipophilic and liquid at room temperature with lower molecular weights than waxes. Where present wax or butter may be present at up to 40% of the oil phase of the emulsion. More preferably the oil phase may contain wax or butter at levels of up to 20% of the oil phase of the emulsion. In an alternative embodiment the oil phase may contain wax or butter at levels of up to 10% of the oil phase of the emulsion.

Preferably the oil phase of the water-in-oil emulsion comprises a silicone oil. Where present, the silicone-containing oil phase preferably comprises an organopolysiloxane oil. The organopolysiloxane oil for use in the composition may be volatile, non-volatile, or a mixture of volatile and non-volatile silicones. The term "non-volatile" as used in this context refers to those silicones that are liquid or gel under ambient conditions and have a flash point (under one atmosphere of pressure) of greater than 100°C. The term "volatile" as used in this context refers to all other silicone oils. Suitable organopolysiloxanes can be selected from a wide variety of silicones spanning a broad range of volatilities and viscosities. Examples of suitable organopolysiloxane oils include polyalkylsiloxanes, cyclic polyalkylsiloxanes, and polyalkylarylsiloxanes.

Preferred for use herein are organopolysiloxanes selected from the group consisting of polyalkylsiloxanes which include low, medium and high molecular weight dimethicone and dimethiconols alkyl substituted dimethicones such as cetyl dimethicone and caprylyl methicone , cyclic organopolysiloxanes having from 3 to 6 silicon atoms are included, for example, octamethylcyclotetrasiloxane, decamethylcyclopentasiloxane, dodecamethylcyclohexasiloxane etc, these are termed cyclomethicones. Also included are silicone resins of the type MQ and T-Propyl also known as trimethylsiloxysilicates and polypropylsilsesquioxane, polyalkylaryl siloxanes, and mixtures thereof. More preferred for use herein are polyalkylsiloxanes and cyclomethicones. Preferred among the polyalkylsiloxanes are dimethicones.

Alternatively, the silicone oil may be a silicone elastomer. Suitable for use herein are silicone elastomers which can be emulsifying or non-emulsifying crosslinked siloxane elastomers or mixtures thereof. No specific restriction exists as to the type of curable organopolysiloxane composition that can serve as starting material for the crosslinked organopolysiloxane elastomer. Examples in this respect are addition reaction-curing organopolysiloxane compositions which cure under platinum metal catalysis by the addition reaction between SiH-containing diorganopolysiloxane and organopolysiloxane having silicon- bonded vinyl groups; condensation-curing organopolysiloxane compositions which cure in the presence of an organotin compound by a dehydrogenation reaction between hydroxyl-terminated diorganopolysiloxane and SiH-containing diorganopolysiloxane and condensation-curing organopolysiloxane compositions which cure in the presence of an organotin compound or a titanate ester. Preferred silicone elastomers include dimethicone crosspolymer and Polysilicone-11.

Preferably the oil phase comprises silicone, and most preferably, a silicone elastomer. Preferably, the emulsion composition includes from 20% to 35%, by weight of the emulsion composition, of the silicone elastomer raw material.

When the composition is a water-in-oil emulsion it preferably comprises an emulsifier. In a preferred embodiment, the composition comprises from 0.1% to 10% emulsifier, more preferably from 0.25% to 7.5%, still more preferably from 0.5% to 5%, emulsifier by weight of the composition. The emulsifier helps disperse and suspend the aqueous water phase within the oil phase.

### Emulsifiers

The pharmaceutical composition may comprise an emulsifier. Suitable emulsifiers include all those suitable for the purpose and known by those skilled in the art for use in skin care products. Preferably these emulsifiers have an HLB value of or less than 14, more preferably from 2 to 14, and still more preferably from 4 to 14.

Silicone emulsifiers are preferred. A wide variety of silicone emulsifiers are useful herein. These silicone emulsifiers are typically organically modified organopolysiloxanes, also known to those skilled in the art as silicone surfactants. Useful silicone emulsifiers include dimethicone copolyols. These materials are polydimethyl siloxanes which have been modified to include polyether side chains such as polyethylene oxide chains, polypropylene oxide chains, mixtures of these chains, and chains comprising moieties derived from both ethylene oxide and propylene oxide. Other examples include alkyl-modified dimethicone copolyols, i.e., compounds which comprise C2-C30 pendant side chains. Still other useful dimethicone copolyols include materials having various cationic, anionic, amphoteric and zwitterionic pendant moieties.

Nonlimiting examples of dimethicone copolyols and other silicone surfactants useful as emulsifiers herein include polydimethylsiloxane polyether copolymers with pendant polyethylene oxide side chains, polydimethylsiloxane polyether copolymers with pendant polypropylene oxide side chains, polydimethylsiloxane polyether copolymers with pendant mixed polyethylene oxide and polypropylene oxide side chains, polydimethylsiloxane polyether copolymers with pendant mixed poly (ethylene) (propylene) oxide side chains, polydimethylsiloxane polyether copolymers with pendant organobetaine side chains, polydimethylsiloxane polyether copolymers with pendant carboxylate side chains, polydimethylsiloxane polyether copolymers with pendant quaternary ammonium side chains; and also further modifications of the preceding copolymers comprising pendant C2-C30 straight, branched, or cyclic alkyl moieties. A particularly preferred emulsifier is PEG/PPG-18/18 dimethicone.

Suitable, cetyl dimethicone copolyol is commercially available as a mixture with polyglyceryl-4 isostearate (and) hexyl laurate or as a mixture with hexyl laurate and polyglyceryl-3 oleate. Other nonlimiting examples of dimethicone copolyols also include lauryl dimethicone copolyol, dimethicone copolyol acetate, diemethicone copolyol adipate, dimethicone copolyolamine, dimethicone copolyol behenate, dimethicone copolyol butyl ether, dimethicone copolyol hydroxy stearate, dimethicone copolyol isostearate, dimethicone copolyol laurate, dimethicone copolyol methyl ether, dimethicone copolyol phosphate, and dimethicone copolyol stearate.

Among the non-silicone-comprising emulsifiers useful herein are various non-ionic and anionic emulsifying agents such as sugar esters and polyesters, alkoxylated sugar esters and polyesters, C1-C30 fatty acid esters of C1-C30 fatty alcohols, alkoxylated derivatives of C1-C30 fatty acid esters of C1-C30 fatty alcohols, alkoxylated ethers of C1-C30 fatty alcohols, polyglyceryl esters of C1-C30 fatty acids, C1-C30 esters of polyols, C1-C30 ethers of polyols, alkyl phosphates, polyoxyalkylene fatty ether phosphates, fatty acid amides, acyl lactylates, soaps, and mixtures thereof. Nonlimiting preferred examples of these non-silicon-comprising emulsifiers include: polyethylene glycol 20 sorbitan monolaurate (Polysorbate 20), polyethylene glycol 5 soya sterol, Steareth-20, Ceteareth-20, PPG-2methyl glucose ether distearate, Ceteth-10, Polysorbate 80, cetyl phosphate, potassium cetyl phosphate, diethanolamine cetyl phosphate, Polysorbate 60, glyceryl stearate, PEG-100 stearate, polyoxyethylene 20 sorbitan trioleate (Polysorbate 85), sorbitan monolaurate, polyoxyethylene 4 lauryl ether sodium stearate, polyglyceryl-4 isostearate, hexyl laurate, steareth-20, ceteareth-20, PPG-2 methyl glucose ether distearate, ceteth-10, diethanolamine cetyl phosphate, glyceryl stearate, PEG-100 stearate, and mixtures thereof.

### Penetration Enhancers

The compositions may further comprise a solvent that acts as a penetration enhancer. The solvent referred to herein increases the solubility of the peptide in the composition, and thus improves the penetration of the composition, particularly the active agent, into the epidermis. This improvement in the transfer of the biologically active agent into the epidermis provides improved efficiency in the delivery of the active agent to the epidermis, particularly, the stratum corneum (SC) of the epidermis. The solvent may be a dimethyl silane. The solvent may be a monosaccharide ether.

The dimethyl silane referred to herein is a water-soluble silicone. It is also clear in water (i.e., without the appearance of oil droplets or a hazy appearance). The dimethyl silane may be or comprise a silicone wax or a silicone oil. A silicone wax refers to a silicone wax with a melting point above 39°C. A silicone oil refers to a siloxane polymer that is a liquid at 25°C and at atmospheric pressure (760 mmHg). The dimethyl silane may be or comprise an ether of dimethyl silane (ether dimethyl silane). Preferably the ether of the dimethyl silane is or comprises a PEG methyl ether dimethyl silane, such as a bis-PEG methyl ether dimethyl silane. Most preferably the dimethyl silane is or comprises bis-PEG- 18 methyl ether dimethyl silane.

The monosaccharide ether referred to herein may be used as a humectant. The monosaccharide ether may be or comprise a triose ether, a pentose ether, a hexose ether or a heptose ether. Preferably the monosaccharide ether is or comprises a hexose ether, such as a glucose ether. The monosaccharide ether may be or comprise an alkoxylated alkyl monosaccharide ether. The alkoxylate may be Cl to C5. The alkyl may be Cl to C5. Preferably, the alkoxylate is C2 and the alkyl is Cl. Preferably, therefore, the alkoxylated alkyl monosaccharide ether is an ethoxylated methyl monosaccharide ether. The alkoxylated monosaccharide ether (e.g., an ethoxylated methyl monosaccharide ether) may be or comprise an alkoxylated alkyl triose ether, an alkoxylated alkyl pentose ether, an alkoxylated alkyl hexose ether or an alkoxylated alkyl heptose ether. Preferably the alkoxylated alkyl monosaccharide ether is or comprises an alkoxylated alkyl hexose ether. More preferably the alkoxylated hexose ether is or comprises an alkoxylated alkyl glucose ether, such as an ethoxylated methyl glucose ether. Preferably, the ethoxylated methyl glucose ether is or comprises methyl- gluceth-20. Methyl-gluceth 20 is an extremely effective humectant.

Thus, the solvent of the composition may be one or more solvents selected from the group consisting of: an ether of dimethyl silane; a monosaccharide ether; and mixtures thereof. The composition according to the invention may further comprise one or more members selected from the group consisting of: solvents (such as glycerides, sorbitan esters, polyglycerin esters, non- silicone fatty compounds, and esters), water-soluble solvents, surfactants (such as sorbitan esters and polyglycerin esters) and mixtures thereof.

The composition may further comprise one or more solvents selected from the group consisting of: glycerides, sorbitan esters, polyglycerin esters, non-silicone fatty compounds, esters, and mixtures thereof.

The term "water-soluble solvent" is interchangeable with the term "water-miscible solvent" and means a compound that is liquid at 25°C and at atmospheric pressure (760 mmHg), and it has a solubility of at least 50% in water under these conditions. In some cases, the water-soluble solvent has a solubility of at least 60%, 70%, 80%, or 90% in water. The solvents can be volatile or non-volatile compounds.

Non-limiting examples of water-soluble solvents may be one or more members selected from the group consisting of glycerin, alcohols (for example, C1-30, C1-15, C1-10, or C1-4 alcohols), organic solvents, polyols (polyhydric alcohols), glycols (e.g., butylene glycol, caprylyl glycol, etc.), and mixtures thereof.

Also suitable are one or more glycols and polyols selected from the group consisting of monomethyl, monoethyl and monobutyl ethers of ethylene glycol, propylene glycol, hexylene glycol or ethers thereof such as, for example, monomethyl ether of propylene glycol, butylene glycol, hexylene glycol, dipropylene glycol as well as alkyl ethers of diethylene glycol, , 1,2,6-hexanetriol, trimethylolpropane, diethylene glycol, triethylene glycol, tetraethylene glycol, pentaethylene glycol, 2-butene-1,4-diol, 2-ethyl-1,3-hexanediol, 2-methyl-2,4-pentanediol, (caprylyl glycol), 1,2- hexanediol, 1,2-pentanediol, and 4-methyl-1,2-pentanediol; ethylene glycol monomethyl ether, ethylene glycol monoethyl ether, ethylene glycol monobutyl ether, ethylene glycol monomethyl ether acetate, diethylene glycol monomethyl ether, diethylene glycol monoethyl ether, diethylene glycol mono-n-propyl ether, ethylene glycol mono- iso-propyl ether, diethylene glycol mono-iso-propyl ether, ethylene glycol mono-n-butyl ether, ethylene glycol mono-t-butyl ether, diethylene glycol mono-t-butyl ether, 1 -methyl- 1-methoxybutanol, propylene glycol monomethyl ether, propylene glycol monoethyl ether, propylene glycol mono-t-butyl ether, propylene glycol mono-n-propyl ether, propylene glycol mono-iso-propyl ether, dipropylene glycol monomethyl ether, dipropylene glycol monoethyl ether, dipropylene glycol mono-n-propyl ether, and dipropylene glycol mono-iso-propyl ether; 2-pyrrolidone, N-methyl-2-pyrrolidone, 1,3-dimethyl-2-imidazolidinone, formamide, acetamide, dimethyl sulfoxide, sorbit, sorbitan, acetine, diacetine, triacetine, sulfolane, and mixtures thereof Also suitable are one or more polyhydric alcohols selected from the group consisting of tripropylene glycol, 1,3-butanediol, 2,3-butanediol, 1,4-butanediol, 3-methyl-1,3-butanediol, 1,5-pentanediol, tetraethylene glycol, 1,6-hexanediol, 2-methyl-2,4-pentanediol, polyethylene glycol, 1,2,4-butanetriol, 1,2,6-hexanetriol, and mixtures thereof. Polyol compounds may also be used e.g., ethoxylation of polyols producing methyl gluceth-20. Ethoxylated water dispersible waxes may be used e.g., Bis PEG-18 methyl ether dimethyl silane.

### Further peptides

The pharmaceutical composition may comprise further peptides. Preferably said additional peptides are selected from the group consisting of dipeptides, tripeptides, additional tetrapeptides, pentapeptides and mixtures thereof. By tripeptides, it is meant compound comprising an uninterrupted sequence of three amino acids. By tetrapeptides, it is meant a compound comprising an uninterrupted sequence of four amino acids and when using further tetrapeptides, the tetrapeptides are referred to as 'additional tetrapeptides'. By pentapeptide it is meant a compound comprising an uninterrupted sequence of five amino acids.

### Dipeptides:

The pharmaceutical compositions may comprise a dipeptide selected from the group consisting of acetyl dipeptide 1 cetyl ester, acetyl dipeptide 3 aminohexanoate, azelaoyl bisdipeptide 10, coumaroyl dipeptide 3, dicetyl dipeptide 9, dipeptide diamino butyroyl benzylamide diacetate, dipeptide 1, dipeptide 10, dipeptide 11, dipeptide 12, dipeptide 15, dipeptide 16, dipeptide 17, dipeptide 18, dipeptide 19, dipeptide 2, dipeptide 20, dipeptide 3, dipeptide 4, dipeptide 5, dipeptide 6, dipeptide 7, dipeptide 8, dipeptide 8 HCL, dipeptide 9, hexanoyl dipeptide 3 norleucine acetate, methyl undecylenoyl dipeptide 16, nicotinoyl dipeptide 22, nicotinoyl dipeptide 23, nicotinoyl dipeptide 24, nicotinoyl dipeptide 26, oleoyl dipeptide 15, palmitoyl dipeptide 10, palmitoyl dipeptide 13, palmitoyl dipeptide17, palmitoyl dipeptide 5 diaminobutyroyl hydroxythreonine, palmitoyl dipeptide 5 diaminohydroxybutyrate, palmitoyl dipeptide 7 and mixtures thereof.

Dipeptides are preferably incorporated into the pharmaceutical composition at a level of from 0.1 to 50000ppm, more preferably from 1 to 5000 ppm, most preferably from 10 to 500ppm.

### Tripeptides:

The pharmaceutical compositions preferably comprise a tripeptide. Said tripeptide may be naturally occurring or of synthetic origin. Suitable tripeptides include tripeptide 1, 2, 3, 4, 5, 6, 7, 8, 9, 10,11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, derivatives thereof and mixtures thereof.

Particularly preferred tripeptides comprise one or more His-based tripeptides. However, another suitable tripeptide may be Arg-Lys-Arg. Particularly preferred tripeptides are based on the structure Gly-His-Lys and its analogs and derivatives thereof. These are collectively known herein as GHK-tripeptides. Indeed, the preferred tripeptide in accordance with this aspect of the invention has this exact sequence of amino acids. Analogs of the preferred tripeptide useful herein include those in which one or more of the three amino acids are reorganized or rearranged within the sequence (e.g., Gly-Lys-His) and/or where no more than two amino acids are substituted (e.g., His-Ala-Orn). However, most preferably, amino acids substituted for Gly include an aliphatic side chain such as, without limitation, beta-Ala, Ala, Val, Leu, Pro, Sarcosine (Sar) and Ile. Most preferred are Ala, Leu and Ile. The most preferable amino acid substituted for Lys or His include those having a side chain that includes, predominantly, a charged nitrogen at a pH of 6, such as, without limitation, Pro, Lys, Arg, His, Desmosine and Isodesmosine. Most preferably, Lys is replaced with Orn, Arg, or Citrulline.

Derivatives are also considered to be encompassed by the term GHK-tripeptides, (and therefore also the more generic term tripeptides). Derivatives of GHK-tripeptides include derivatives of the substituted and rearranged tripeptides described herein. These derivatives include, inter alia, acyl-derivatives, which are tripeptides substituted with one or more straight-chain or branched-chain, long or short chain, saturated or unsaturated, substituted with a hydroxy, amino, acyl amino, sulfate or sulfide group, or unsubstituted, which can be derived from acetic acid, capric acid, lauric acid, myristic acid, octanoic acid, palmitic acid, stearic acid, behenic acid, linoleic acid, linolenic acid, lipoic acid, oleic acid, isostearic acid, elaidoic acid, 2-ethylhexaneic acid, coconut oil fatty acid, tallow fatty acid, hardened tallow fatty acid, palm kernel oil fatty acid, lanolin fatty acid and the like. Preferable examples of the acyl group include an acetyl group, a palmitoyl group, an elaidoyl group, a myristyl group, a biotinyl group and an octanoyl group. These may be substituted or unsubstituted. When substituted, they are preferably substituted with hydroxyl or sulphur comprising groups such as, without limitation SO₃H, SH or S-S.

His-based tripeptides include at least one histidine amino acid. The other two amino acids in the sequence may be the same or different. Thus, contemplated are, without limitation, His-Xaa-Xaa, His-Xaa-Xbb, His-Xbb-Xaa, Xbb-His-Xbb, Xbb-His-Xaa, Xaa-His-Xbb, Xaa-Xaa-His, Xaa-Xbb-His, Xbb-Xaa-His and Xbb-Xbb-His, where Xaa and Xbb are two different amino acids, although either can be His. Preferably, at least one of the other amino acids is Gly, beta-Ala, Ala, Val, Leu, Pro, Sarcosine (Sar) or Ile. Preferably, at least one of the other amino acids is Pro, Lys, Arg, His, Desmosine and Isodesmosine. Most preferably, Lys is replaced with Orn, Arg, or Citrulline.

Derivatives are also considered to be encompassed by the term His-based, (and therefore also the more generic term tripeptides). These derivatives include, inter alia, acyl-derivatives, which are tripeptides substituted with one or more straight-chain or branched-chain, long or short chain, saturated or unsaturated substituted or unsubstituted acyl group(s) having from 1 to 29 carbon atoms. The acyl groups which can be used are the same as those described for the GHK-tripeptides.

Particularly preferred embodiments of tripeptides include N-Acyl-Gly-His-Lys and most preferably, N-Palmitoyl-Gly-His-Lys. Preferred commercially available tripeptide and tripeptide derivative comprising compositions include Biopeptide-CL from SEDERMA, Maxilip(R) from SEDERMA, Biobustyl(R) from SEDERMA.

The tripeptides where included are preferably incorporated into the pharmaceutical composition in amounts of from 0. 10ppm to 10,000ppm, preferably from 0. 50ppm to 5,000ppm, more preferably from 1ppm to 1000ppm, and most preferably from 1ppm to 500ppm. These are again based on a % w/w basis. Thus 100,000ppm is 10% by weight of the emulsion.

### Additional tetrapeptides:

The pharmaceutical composition may comprise an additional tetrapeptide. These may be one or more rigin-based tetrapeptides, one or more ALAMCAT-tetrapeptides or mixtures thereof. These tetrapeptides may be naturally occurring or of synthetic origin. Suitable tetrapeptides for use in the present composition include those selected from the group consisting of well-known tetrapeptide 1, 2, 3, 4, 5, 6, 7, 8, 9, 10, 11, 12,13, 14, 15, 16, 17, 18, 19 ,20, 21, 22, 23, 24, 25, 26, 27, 28, 29, 30, 34, 35, derivatives thereof and mixtures thereof.

Rigin-based tetrapeptides are based on the structure Gly-Gln-Pro-Arg (Rigin) and include its analogs and derivatives thereof. Rigin is an additional tetrapeptide. Analogs of the tetrapeptide rigin useful include those in which one or more of the four amino acids are reorganized or rearranged within the sequence and/or where no more than two of the amino acids are substituted (e.g., Ala-Gln-Thr-Arg. More preferably, at least one of the amino acids within the sequence is Pro or Arg and most preferably the tetrapeptide includes both Pro and Arg although their order and position may vary. The amino acid substitutions can be from amongst any amino acid as defined herein. Particularly preferred rigin-based tetrapeptides include Xaa-Xbb-Arg-Xcc, Xaa-Xbb-Xcc-Pro, Xaa-Xbb-Pro-Arg, wherein Xaa-Xbb-Pro-Xcc, Xaa-Xbb-Xcc-Arg, Xaa, Xbb and Xcc may be the same or different and selected from the following Xaa is Gly or the amino acids that may be substituted therefore, Xbb is Gln or the amino acids that may be substituted therefore and Xcc may be Pro or Arg or the amino acids substituted therefore. The most preferable amino acids substituted for Gly include an aliphatic side chain such as, without limitation, beta-Ala, Ala, Val, Leu, Pro, Sarcosine (Sar) and Ile. The most preferable amino acids substituted for Gln include a side chain that includes an amine group that is predominantly uncharged at neutral pH (pH 6-7) such as, without limitation, Asn, Lys, Orn, 5-hydroxyproline, Citrulline and Canavanine. When Arg is substituted, it is preferably replaced with an amino acid having a side chain that includes, predominantly, a charged nitrogen at a pH of 6, such as, without limitation, Pro, Lys, His, Desmosine and Isodesmosine.

Derivatives are also considered to be encompassed by the term rigin-base tetrapeptides, (and therefore also the more generic term tetrapeptides). Derivatives include derivatives of the substituted and rearranged rigin-based tetrapeptides described herein. These derivatives include, inter alia, acyl-derivatives, which are tetrapeptides substituted with one or more straight-chain or branched-chain, long or short chain, saturated or unsaturated, substituted with a hydroxy, amino, amino acyl, sulfate or sulfide group or unsubstituted having from 1 to 29 carbon atoms. N-acyl-derivatives include those acyl groups which can be derived from acetic acid, capric acid, lauric acid, myristic acid, octanoic acid, palmitic acid, stearic acid, behenic acid, linoleic acid, linolenic acid, lipoic acid, oleic acid, isostearic acid, elaidoic acid, 2-ethylhexaneic acid, coconut oil fatty acid, tallow fatty acid, hardened tallow fatty acid, palm kernel oil fatty acid, lanolin fatty acid and the like. Preferable examples of the acyl group include an acetyl group, a palmitoyl group, an elaidoyl group, a myristyl group, a biotinyl group and an octanoyl group. These may be substituted or unsubstituted. When substituted, they are preferably substituted with hydroxyl or sulphur comprising groups such as, without limitation SO₃H, SH or S-S.

Derivatives are also considered to include peptide-divalent ion complexes. Cu²⁺-peptide derivatives are preferred as this may provide increased biological effect compared to the peptide alone.

ALAMCAT tetrapeptides are tetrapeptides which include at least one amino acid including an aliphatic group comprising side chain. These amino acids include, without limitation, Gly, beta-Ala, Ala, Val, Leu, Sarcosine (Sar) and Ile. These tetrapeptides also include at least one amino acid including at least one NH+ comprising side chain. These amino acids include a side chain that has an amine group that is predominantly uncharged at neutral pH (pH 6-7) such as, without limitation, Gln, Asn, Lys, Orn, 5-hydroxyproline, Citrulline and Canavanine. The ALAMCAT-tetrapeptides also include at least one amino acid having at least one side chain including at least one cationic amine (predominant species is charged such as NH₃⁺, NH₂⁺, etc.-basic amino acids which are positively charged at pH 6.0). These amino acids include, without limitation, Pro, Arg, Lys, His, Desmosine and Isodesmosine. The remaining amino acid can be any amino acid, but is preferably one comprising an aliphatic group, pendant amino group or pendant cationic group. Derivatives are also considered to be encompassed by the term ALAMCAT-tetrapeptides, (and therefore also the more generic term tetrapeptides). These derivatives include, inter alia, acyl-derivatives, which are tetrapeptides substituted with one or more straight-chain or branched-chain, substituted or unsubstituted long or short chain, saturated or unsaturated acyl group(s) having from 1 to 29 carbon atoms. The acyl groups which can be used are the same as those described for the rigin-based tetrapeptides.

Preferred embodiments include Peptide E, arg-ser-arg-lys, N-acyl-Gly-Gln-Pro-Arg peptides, most preferably N-palmitoyl-Gly-Gln-Pro-Arg.

Preferred commercially available sources of tetrapeptides include RIGIN, EYELISS, Haloxyl, and MATRIXYL 3000, which comprise between 50 to 500 ppm of palmitoyl-Gly-Gln-Pro-Arg, and other ingredients, such as peptides, chalcones and an excipient, commercially available from SEDERMA, France. Tego Pep 417 available from Evonik. These may be used to produce compositions by adding thereto at least one tripeptide as described herein.

The additional tetrapeptides when used are preferably incorporated in to the pharmaceutical composition in amounts from 0.1 ppm (0.00001% w/w also referred to herein as "weight percent", "weight %" or simply by weight) to 10,000 ppm (1.0% w/w), preferably from 0.5 ppm to 1000 ppm (0.1% w/w), and most preferably from 1 ppm to 500ppm (0.05% w/w) by weight of the composition.

The combination of tripeptides and additional tetrapeptides, can be particularly preferred. When present, the preferred ratio of additional tetrapeptide to tripeptide, or indeed the ratio of molecules having four amino acids to those having three amino acids can range from 100:1 to 1:100; more preferably from 50:1 to 1:50, even more preferably from 30:1 to 1:30 and even more preferably between 10:1 to 1:10. Most preferably, the ratio of additional tetrapeptide to tripeptide ranges from between 3:1 to 1:3. These ratios are on a weight basis (% w/w-e.g. mg of pure peptide per Kilogram in the final formulation). In a particularly preferred embodiment, the amount of tripeptide used is greater than the amount of additional tetrapeptide used when considered in terms of their amounts in parts per million, again based on overall weight of the composition. In a particularly preferred embodiment, the pharmaceutical composition comprises an additional tetrapeptide of the sequence Gly-Gln-Pro-Arg, its analogs and derivatives in combination with one or more tripeptide of the sequences Gly-His-Lys, its analogs and derivatives.

### Pentapeptides:

The compositions may optionally comprise a pentapeptide, derivatives of pentapeptides, and mixtures thereof. As used herein, "pentapeptides" refers to both the naturally occurring pentapeptides and synthesized pentapeptides. Also, useful herein are naturally occurring and commercially available compositions that comprise pentapeptides. Suitable pentapeptides are those selected from the group consisting of pentapeptide1, 4, 5, 6, 7, 8, 9, 10, 11,12, 13,14, 15,16, 17,18, 19, 21, 22, 23, 24, 25, 26, 28, 29, 30, 31, 33, 34, 35, 36, 38, 39, derivatives thereof and mixtures thereof.

Suitable pentapeptides for use herein are the pentapeptide, lys-thr-thr-lys-ser, arg-asp-lys-tyr-val (pentapeptide-1) and derivatives thereof. A preferred commercially available pentapeptide derivative-comprising composition is Matrixyl which comprises 100 ppm of palmitoyl-lys-thr-thr-lys-ser and is commercially available from Sederma, France.

The pentapeptides when used are preferably incorporated into the pharmaceutical composition in amounts from 0.1 ppm (0.00001% w/w also referred to herein as "weight percent", "weight %" or simply by weight) to 10,000 ppm (1.0% w/w), preferably from 0.5 ppm to 1000 ppm (0.1% w/w), and most preferably from 1 ppm to 500ppm (0.05% w/w) by weight of the composition.

### Hexapeptides:

The compositions may optionally comprise a hexapeptide, derivatives of hexapeptides, and mixtures thereof. As used herein, "hexapeptides" refers to both the naturally occurring hexapeptides and synthesized hexapeptides. Also, useful herein are naturally occurring and commercially available compositions that comprise hexapeptides.

The hexapeptides when used are preferably incorporated into the pharmaceutical composition in amounts from 0.1 ppm (0.00001% w/w also referred to herein as "weight percent", "weight %" or simply by weight) to 10,000 ppm (1.0% w/w), preferably from 0.5 ppm to 1000 ppm (0.1% w/w), and most preferably from 1 ppm to 500ppm (0.05% w/w) by weight of the composition.

### Matrix metalloproteinase inhibitors (MMPi)

The term "matrix metalloproteinase inhibitor" relates to all molecule and/or plant or bacterial extracts having an inhibitory activity on at least one of the matrix metalloproteinases expressed or synthetized by or in the skin. The family of the matrix metalloproteinases is formed of several well-defined groups on the basis of their resemblance regarding structure and substrate specificity (Woessner J. F. 1991, Faseb Journal, vol. 5,-, 2145). Among these groups, there are collagenases able to degrade fibrillar collagens (MMP-1 or interstitial collagenase, MMP-8 or neutrophil collagenase, MMP- 13 or collagenase 3, MMP-18 or collagenase 4), gelatinases degrading type IV collagen or other denatured collagen form (MMP-2 or A gelatinase (72 kDa), MMP-9 or B gelatinase (92 kDa)), stromelysins (MMP-3 or stromelysin 1, MMP- 10 or stromelysin 2, MMP-11 or stromelysin 3) whose broad spectrum of activity targets proteins of the dermal extracellular matrix such as glycoproteins (fibronectin, laminin), proteoglycanes etc., matrilysin (MMP-7), metalloelastase (MMP- 12) or metalloproteinases (MMP- 14, MMP- 15, MMP- 16 and MMP- 17). Metalloproteinases (MMPs) are proteases that use a metal, (mostly zinc) coordinated to 3 cysteine residues and to a methionine in their active site, that degrade macromolecular components of the dermal extracellular matrix and of basal layers at neutral pH (collagen, elastin, etc ...). This group of enzymes is inactivated by metal chelators. The principal activity regulators of MMPs are the tissue inhibitors of metalloproteinases or TIMPs such TIMP-I, TIMP-2, TIMP-3 and TIMP-4 (Woessner J. F., Faseb Journal, 1991). Furthermore, MMP expression is also regulated by growth factors, cytokines, oncogene products (ras, jun), or also matrix constituents.

The term "matrix metalloproteinase inhibitors " according to the present invention means all molecules able to reduce the MMP's activity regarding the gene expression (transcription and translation) or regarding the activation of the zymogen form of the MMP, or else regarding the local control of active forms. Furthermore, the metalloproteinase inhibitors according to the present invention can also be MMP-1 inhibitors of natural or synthetic origin. The terms "natural origin" or "synthetic origin" mean both a metalloproteinase inhibitor at a pure state or in solution at different concentrations, but natural origin termed inhibitors are obtained by different extraction methods from a natural element (for example lycopene from a tomato) whereas the inhibitors of synthetic origin are all obtained via chemical synthesis.

Preferred MMPi are selected from the group consisting of retinoid, N-acetyl cysteine, glutathione, 2-furildioxime, vitamin C, flavones, isoflavones, hydrolysed rice protein, alfalfa extract, white lupin, zizyphus jujube extract, dihydroxy methyl chromone, kudzu extract, vitis vinifera extract, Oenothera biennis extract Anogeissus leiocarpus extract and mixtures thereof.

Where present, MMPi are present at a level of from 0.01% to 10%, more preferably 0.1% to 5% and most preferably from 0.5% to 2.5% by weight of the pharmaceutical composition.

### Skin Conditioning Agent

The compositions may optionally comprise a skin conditioning agent. Said skin conditioning agents may preferably be selected from the group consisting of humectants, emollients, moisturisers, or mixtures thereof. Where present, they are preferably present at a level of from 0.01% to 20%, more preferably from 0. 1 % to 10%, most preferably from 0.5% to 7% by weight of the pharmaceutical composition.

Preferred skin conditioning agents are selected from the group consisting of guanidine, urea, glycolic acid and glycolate salts, salicylic acid, lactic acid and lactate salts, aloe vera, shea butter, polyhydroxy alcohols, such as sorbitol, mannitol, xylitol, erythritol, glycerol, hexanetriol, butanitriol, (di) propylene glycol, butylene glycol, hexylene glycol, polyethylene glycol, sugars (e.g. fructose, glucose, xylose, honey, mannose, xylose), gluconodeltalactone, and starches and their derivatives, pyrrolidone, carboxylic acid, hyaluronic acid and salts thereof, lactamide monoethanolamine, acetamide monoethanolamine, panthenol, allantoin and mixtures thereof.

More preferably said skin conditioning agent is selected from the group consisting of glycerine, arabinogalactan, butylene glycol, hyaluronic acid, shea butter, propylene glycol, ethylhexyl glycerine, hyaluronate and mixtures thereof.

### Antioxidant Agent

The compositions may optionally comprise an antioxidant agent. Suitable antioxidant agents may include: a) ascorbic acid its salts, esters, glucosides and glucosamines, particularly sodium ascorbyl phosphate, magnesium ascorbyl phosphate, ascorbyl palmitate and ethyl ascorbic acid b) vitamin E (tocopherol) and its esters, particularly tocopheryl acetate, as well as Dimethyl methoxy chromanol which is a synthetic analogue of gamma tocopherol, available from Lipotec S.A. polygon Industrial Camri Ral, under the tradename Lipochroman-6 c) herbal extracts, particularly gingko biloba, such as that available under the trade name "Gingko Biloba Leaf Powder" from Univar PLC, morus alba, such as that available under the trade name "Mulberry Concentrate" from Solabia, origanum vulgare, such as that available under the trade name "Pronalen Origanum HSC" from S Black Ltd, panax ginseng, such as that available under the trade name "Panax ginseng 1.1 extract 4294"from S Black Ltd or "Phytexcell Panax ginseng" available from Croda Chemicals Ltd, birch extract such as those available from Cosmetochem (U. K.) Ltd under the trade names "Super Herbasol Extract Birch" and "HP Herbasol Betula" and those available from Blagden Chemicals under the tradenames "Phytelene of Birch" and "Aqueous Spray Dried Birch", camellia sinensis, such as that available under the trade name "Herbal Extract Green Tea 75% Solids" from Nichimen Europe, rosmarinus officinalis, such as that available under the trade name "Pronalen Rosemary" from S. Black, Acerola cherry powder, such as that available as Acerola PE from Gee Lawson, Emblica extract sold under the tradename Emblica ^{™} by Merck Speciality chemicals, and Grape Seed oil, such as that available from Chesham Chemicals Limited.

The amounts of antioxidant agents used in the pharmaceutical composition are expressed as dry weights, as understood by a man skilled in the art. The total amount of antioxidant agents optionally present in the composition may range from 0.005% to 10% by weight, preferably 0.5% to 5%, most preferably 0.2% to 1.5% by weight of the composition.

Particularly preferred synergistic combinations of antioxidant agents suitable for inclusion in the pharmaceutical composition are selected from the group consisting of: i) panax ginseng, morus alba and magnesium ascorbyl phosphate; ii) panax ginseng, morus alba and sodium ascorbyl phosphate; iii) panax ginseng, morus alba and rosmarinus officinalis; iv) ginkgo biloba, phyllanthus emblica and Dimethylmethoxy chromanol; v) morus alba, camellia sinensis and dimethylmethoxy chromanol; vi) morus alba, camellia sinensis and tocopheryl acetate; vii) panax ginseng, morus alba and origanum vulgare, viii) camellia sinensis, tocopheryl acetate and dimethylmethoxychromanol, viv) morus alba, tocopheryl acatete and dimethylmethoxychromanol.

In these preferred combinations (a) the panax ginseng is preferably present in an amount of 0.005% to 0.1%, more preferably 0.01% to 0.05% by weight of the composition; (b) the morus alba is preferably present in an amount of 0.0005% to 0.01%, more preferably 0.001% to 0.005% by weight of the composition; (c) the sodium, magnesium ascorbyl phosphate or ethyl ascorbic acid is preferably present in an amount of 0.05% to 2.5%, preferably 0.1% to 2%, most preferably 0.15% to 1.5% by weight of the composition;(d) the rosmarinus officinalis or origanum vulgare or phyllanthus emblica is preferably present in an amount of 0.01% to 0.5%, more preferably 0.05% to 0.2% by weight of the composition e) the dimethylmethoxy chromanol is preferably present in an amount of 0.0005 % to 0.1%, more preferably from 0.005% to 0.05% by weight of the composition ; f) the camellia sinensis is preferably present in an amount of 0.005 % to 0.2%, more preferably from 0.01 % to 0.1 % and the g) Tocopherol acetate is preferably present in an amount of 0.01 to 0.5%, more preferably from 0.05% to 0.25%

### Vitamins

The pharmaceutical compositions may comprise one or more vitamins. The compositions may comprise ascorbates, for example vitamin C, vitamin C derivatives, ascorbic acid, ascorbyl glucoside, ascorbyl palmitate, magnesium ascorbyl phosphate, sodium ascorbyl phosphate and ethyl ascorbic acid. The composition may comprise vitamin B, vitamin B derivatives, vitamin B1 to vitamin B12 and their derivatives. In a further embodiment the composition comprising the Vitamin B3 derivative niacinamide.

In an alternative embodiment of the present invention the pharmaceutical composition comprises vitamin K, vitamin K derivatives, vitamin H, vitamin D, vitamin D derivatives and mixtures thereof. In an alternative embodiment of the present invention the pharmaceutical composition comprises vitamin E, vitamin E derivatives such as tocopherol and tocopheryl acetate, and provitamins thereof, such as panthenol and mixtures thereof.

In a further embodiment the present pharmaceutical composition comprises retinoid compounds, including retinoic acid, retinaldehyde, retinol and derivatives thereof. In one embodiment the pharmaceutical composition comprises retinyl palmitate, retinyl acetate, retinyl retinoate, retinyl proprionate, retinyl ascorbate, retinyl linoleate, retinyl retinoate, retinyl sunflowerseedate and mixtures thereof.

The vitamin compounds may be included as the substantially pure material, or as an extract obtained by suitable physical and/or chemical isolation from natural (e. g. plant) sources. In one embodiment, when vitamin compounds are present in the compositions of the instant invention, the emulsion compositions comprise from about 0.0001 % to 50%, more preferably from 0.001% to 10%, still more preferably from 0.01% to 8%, and still more preferably from 0.1% to 5%, by weight of the composition, of the vitamin compound.

### Salicylic Acid Compound

The pharmaceutical compositions may comprise a salicylic acid compound, its esters, its salts, or combinations thereof. In one embodiment of the compositions, the salicylic acid compound preferably comprises from 0.0001 % to 25%, more preferably from 0.001% to 15%, even more preferably from 0.01% to 10%, still more preferably from 0.1% to 5%, and even more preferably from 0.01% to 2%, more preferably 0. 1% to 2% by weight of the composition, of salicylic acid.

### Sunscreen

The pharmaceutical compositions may optionally comprise a sunscreen component. The sunscreen may comprise organic or inorganic sun filters or a combination of the two. Suitable inorganic sun filters include those selected from the group consisting of microfine titanium dioxide, microfine zinc oxide, boron nitride and mixtures thereof. Suitable organic sunscreens include those selected from the group consisting of: a) p-aminobenzoic acids, their esters and derivatives (for example, 2ethylhexyl p-dimethylaminobenzoate), b) methoxycinnamate esters (for example, 2-ethylhexyl p-methoxycinnamate, 2-ethoxyethyl p-methoxycinnamate or a, p-di- (p-methoxycinnamoyl)-a'- (2ethylhexanoyl)-glycerin, c) benzophenones (for example oxybenzone), d) dibenzoylmethanes such as 4- (tert-butyl)-4'-methoxydibenzoylmethane, e) 2-phenylbenzimidazole-5 sulfonic acid and its salts, f) alkyl-ss, ss-diphenylacrylates for example alkyl a-cyano-ss, ss-diphenylacrylates such as octocrylene, g) triazines such as 2,4,6-trianilino- (p-carbo-2-ethylhexyl-1-oxi)-1, 3,5 triazine, h) camphor derivatives such as methylbenzylidene camphor and i) mixtures thereof. Other preferred sunscreen ingredients include those selected from the group consisting of homosalate, Ethylhexyl salicylate, Diethylhexylbutamido triazone, Bis-ethylhexyloxyphenol methoxyphenyl triazine, Diethylamino hydroxybenzoyl hexyl benzoate, Butyl methoxydibenzoylmethane, Methylene bis-benzotriazoyl tetramethylbutylphenol, Polysilicone-15 and mixtures thereof. A sunscreen agent is optionally present in an amount from 0.1 to 10% by weight of the composition.

### Other Optional Ingredients

The pharmaceutical composition may comprise a thickener. The thickener may be present in an amount of from 0.5 to 10% by weight of the composition. Non-limiting examples of various types of thickeners include:

### a. Carboxylic Acid Polymers

These polymers are crosslinked compounds containing one or more monomers derived from acrylic acid, substituted acrylic acids, and salts and esters of these acrylic acids and the substituted acrylic acids, wherein the crosslinking agent contains two or more carbon-carbon double bonds and is derived from a polyhydric alcohol. Examples of commercially available carboxylic acid polymers useful herein include the carbomers, which are homopolymers of acrylic acid crosslinked with allyl ethers of sucrose or pentaerythritol. The carbomers are available as the CARBOPOL 900 series from Lubrizol (e.g., Carbopol^{®} 980). In addition, other suitable carboxylic acid polymeric agents include Ultrez^{®} 10 or Ultrez^{®} 30 (Lubrizol) and copolymers of C10-30 alkyl acrylates with one or more monomers of acrylic acid, methacrylic acid, or one of their short chain (i.e., C1-4 alcohol) esters, wherein the crosslinking agent is an allyl ether of sucrose or pentaerythritol.

These copolymers are known as acrylates/C10-C30 alkyl acrylate crosspolymers and are commercially available as Carbopol^{®} 1382, Ultrez^{®} 21, Pemulen TR-1, Pemulen TR-2 and Pemulen EZ-4U from Lubrizol. In other words, examples of carboxylic acid polymer thickeners useful herein are those selected from carbomers, acrylates/C10-C30 alkyl acrylate crosspolymers, and mixtures thereof.

### b. Crosslinked Polyacrylate Polymers

The compositions of the present disclosure can optionally contain crosslinked polyacrylate polymers useful as thickeners or gelling agents including both cationic and nonionic polymers. Examples of crosslinked polyacrylate polymers include Polyacrylate crosspolymer-6.

### c. Polyacrylamide Polymers

The compositions of the present disclosure can optionally contain polyacrylamide polymers, especially nonionic polyacrylamide polymers including substituted branched or unbranched polymers. Among these polyacrylamide polymers is the nonionic polymer given the CTFA designation polyacrylamide and isoparaflin and laureth-7, available under the Tradename Sepigel 305 from Seppic. Other polyacrylamide polymers useful herein include multi-block copolymers of acrylamides and substituted acrylamides with acrylic acids and substituted acrylic acids. Commercially available examples of these multi-block copolymers include Hypan 5R150H, 55500V, SSSOOW, SSSA100H, from Lipo Chemicals, Inc. The compositions may also contain thickening and texturising gels of the type as exemplified by the product range 40 called Lubrajel^{®} from United Guardian. These gels have moisturizing, viscosifying, stabilizing properties.

### d. Polysaccharides

A wide variety of polysaccharides can be useful herein. "Polysaccharides" refer to gelling agents that contain a45 backbone of repeating sugar (i.e., carbohydrate) units. Nonlimiting examples of polysaccharide gelling agents include those selected from the group consisting of cellulose, carboxymethylhydroxyethylcellulose, cellulose acetate propionate carboxylate, hydroxyethylcellulose, hydroxyethyl ethylcellulose, hydroxypropylcellulose, hydroxypropyl methylcellulose, methyl hydroxyethylcellulose, microcrystalline cellulose, sodium cellulose sulfate, and mixtures thereof. Also, useful herein are the alkyl-substituted celluloses.

### e. Gums

Other thickening and gelling agents useful herein include materials which are primarily derived from natural sources. Nonlimiting examples of these gelling agent gums include acacia, agar, algin, alginic acid, ammonium alginate, amylopectin, calcium alginate, calcium carrageenan, carnitine, carrageenan, dextrin, gelatin, gellan gum, guar gum, guar hydroxypropyltrimonium chloride, hectorite, hyaluronic acid, hydrated silica, hydroxypropyl chitosan, hydroxypropyl guar, karaya gum, kelp, locust bean gum, natto gum, potassium alginate, potassium carrageenan, propylene glycol alginate, scierotiurn gum, sodium carboxymethyl dextran, sodium carrageenan, tragacanth gum, xanthan gum, biosacharide gum, and mixtures thereof. Additional examples of water-soluble thickeners include water-soluble natural polymers, water-soluble synthetic polymers, clay minerals and silicic anhydride. Non-limiting examples of water-soluble natural polymers include gum arabic, tragacanth gum, karaya gum, guar gum, gellan gum, taragum, locust bean gum, tamarind gum, sodium alginate, alginic acid propyleneglycol ester, carrageenan, farcelluran, agar, high-methoxy pectin, low-methoxy pectin, xanthine, chitosan, starch, fermentation polysaccharide (for example, xanthan gum, pullulan, carciran, dextran), acidic heteropolysaccharide derived from callus of plants belonging to Polyantes sp. (for example, tuberous polysaccharide), proteins (for example, sodium casein, gelatin, albumin), chondroitinsulfate, and hyaluronic acid. Non-limiting examples of water-soluble synthetic polymers include polyvinyl alcohol, sodium polyacrylate, sodium polymethacrylate, polyacrylic acid glycerin ester, carboxyvinyl polymer, polyacrylamide, polyvinyl pyrrolidone, polyvinyl methylether, polyvinyl sulfone, maleic acid copolymer, polyethylene oxide, polydiallyl amine, polyethylene imine, water soluble cellulose derivatives (for example, carboxymethyl cellulose, methyl cellulose, methylhydroxypropylcellulose, hydroxyethyl cellulose, hydroxypropyl cellulose, cellulose sulfate sodium salt),

Additionally, the one or more thickeners may include polymeric thickeners selected from the group consisting of ammonium polyacryloyldimethyl taurate, ammonium acryloyldimethyltaurate/VP copolymer, sodium polyacrylate, acrylates copolymers.

Preferred thickeners are selected from the group consisting of xanthan gum, ammonium acryloyldimethyltaurate/vinyl pyrrolidone copolymer, dimethicone crosspolymer, carbomer, hydroxyethyl cellulose, polyacrylamide, polyacrylate crosspolymer-6, and mixtures thereof.

The compositions may also optionally comprise one or more of the following optional ingredients. Preservatives may be added to the emulsion such as 2-bromo2-nitropropane-1,3-diol (bronopol, which is available commercially under the trade name Myacide RTM), benzyl alcohol, diazolidinyl urea, imidazolidinyl urea, methyl paraben, phenoxy ethanol, ethyl paraben, propyl paraben, sodium methyl paraben, sodium dehydroacetate, polyhexamethylenebiguanide hydrochloride, isothiazolone and sodium propyl paraben, suitably in an amount of from 0.01% to 10% by weight of the emulsion.

Sequestering agents may be added to the emulsion composition, such as ethylenediamine tetraacetic acid and salts thereof, suitably in an amount of from 0.005% to 0.5% by weight of the composition.

The composition may also include waxes such as cocoa butter suitably in an amount of from 1% to 99% by weight of the composition.

The composition may also comprise suitable, pharmaceutically acceptable diluents, carriers and/or propellants such as dimethyl ether.

The composition may also include pearlising agents such as stearic monoethanolamide and/or mica, suitably in an amount of from 0.01% to 10% by weight of the composition.

Perfumes may be added suitably in an amount of from 0.01% to 2% by weight of the composition, as may water soluble dyes such as tartrazine, suitably in an amount of from a trace amount (such as 1 × 10-5 %) to 0.1 % by weight of the composition.

The composition may also include pH adjusting agents such as sodium hydroxide, aminomethyl propanol, triethanolamine, suitably in an amount of from 0.01 % to 10% by weight of the composition. The composition may be buffered by means well known in the art, for example by use of buffer systems comprising succinic acid, citric acid, lactic acid, and acceptable salts thereof, phosphoric acid, mono-or disodium phosphate and sodium carbonate. Suitably, the composition may have a pH between 3 and 10, preferably between 4 and 8.

### Uses and methods of use

The composition for use in a method of treating a skin wound of a subject may be applied topically to the skin wound.

The skin wound may be an abrasion (such as a linear or scratch abrasion, patterned abrasions and grazed or brush abrasions), laceration (such as a split laceration, torn laceration, stretch laceration, perforated laceration, blast laceration, cut laceration, crush laceration or de-gloved laceration), burn wound (such as a thermal burn, radiation burn, chemical burn or electrical burn), surgical incision, pressure injury, skin tear, arterial ulcer, venous ulcer, or bruise.

The present invention also relates to a method of treating a wound in the skin of a subject. The method comprises administering to a wound in the skin of said subject the pharmaceutical composition (e.g. in a therapeutically effective amount) according to the present invention.

### Examples

The present invention is further described by the following examples.

### Examples of tetrapeptide synthesis

The tetrapeptides of the present disclosure with generic formulation pal-X¹X²X³X⁴-OH are prepared by peptidic synthesis. In a first step the N-terminal of X⁴ is coupled with a resin via the terminal acid functionality in the presence of a coupling agent. The N terminal amine is then reacted with the next amino acid in the sequence X³ in the presence of a coupling agent. The same process is repeated until the required sequence is obtained and a suitable C terminal functionality added. Suitable coupling agents include DCC (dicyclohexylcarbodiimide)/NHS (N-hydroxysuccinimide) or HBTU (2-(1H-benzotriazole-1-yl)-1,1,3,3-tetramethyluronium hexafluorophosphate)/HOBT (1-hydroxy-benzotiiazole)). The resulting peptide is then cleaved from the resin in an acidic medium and after precipitation, washing and drying, the peptide is obtained in solid form.

### Excipient composition

Tetrapeptides may be provided in an excipient composition.

To form a tetrapeptide excipient composition, the tetrapeptide is combined with an oil-based matrix comprising a fatty acid ester, stirred and heated until the tetrapeptide is completely solubilised, and the excipient composition is clear. In the following example compositions, 1200ppm of tetrapeptide is used in the excipient composition.

### Example composition

### Representative water-in-oil emulsion composition

| Material | % w/w |
|---|---|
| Dimethicone | 13.83 |
| Water | 37.11 |
| Glycerin | 5.00 |
| Dimethicone crosspolymer & Dimethcone | 32.31 |
| Butylene glycol | 2.60 |
| PEG/PPG-18/18 dimethicone & Polyglyceryl -4 isostearate & Hexyl laurate | 3.00 |
| Cetyl PEG/PPG-10/1 dimethicone | 2.00 |
| Magnesium sulphate | 0.60 |
| Phenoxyethanol & Methylparaben & Ethylparaben | 0.55 |
| Peptide excipient composition comprising propanediol & Pentylene glycol & Decyl glucoside & Water excipient composition | 3.00 |

### Method of manufacture

1. In the main vessel add Dimethicone, Dimethicone crosspolymer, PEG/PPG-18/18 dimethicone & polyglyceryl-4 isostearate & hexyl laurate and Cetyl PEG/PPG-10/1 dimethicone to make the oil phase.
2. Separately weigh out water, magnesium sulphate, glycerine, phenoxyethanol & methylparaben & ethylparaben and peptide & propanediol & pentylene glycol & decyl glucoside & water stir until solids are dissolved to make the water phase.
3. Add the water phase to the oil phase slowly with constant stirring at high speed (creating a vortex). Continue stirring for 5 minutes.
4. Homogenise the product for 5 minutes at 3500 rpm using a Silverson mixer or equivalent.

## Claims

1. A pharmaceutical composition for use in a method of treating a wound in the skin of a subject, the pharmaceutical composition comprising a tetrapeptide having the amino acid sequence U-XXGD-Z wherein G is used to denote amino acid Glycine and D is used to denote amino acid Aspartic acid, as per the internationally recognised single letter code for amino acids; X denotes an amino acid selected from the group consisting of Glutamic acid (E), Lysine (K), Leucine (L), Alanine (A), Isoleucine (I), Arginine (R) and mixtures thereof; at the N-terminal end, U is selected from the group consisting of H, -CO-R¹, - SO₂-R¹ or a biotinyl group; at the C-terminal end, Z is selected from the group consisting of OH, OR¹, NHR¹ or NR¹R²; R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N.

2. A method of treating a wound in the skin of a subject, wherein the method comprises administering a pharmaceutical composition to the wounded skin of a subject, the pharmaceutical composition comprising a tetrapeptide having the amino acid sequence U-XXGD-Z wherein G is used to denote amino acid Glycine and D is used to denote amino acid Aspartic acid, as per the internationally recognised single letter code for amino acids; X denotes an amino acid selected from the group consisting of Glutamic acid (E), Lysine (K), Leucine (L), Alanine (A), Isoleucine (I), Arginine (R) and mixtures thereof; at the N-terminal end, U is selected from the group consisting of H, -CO-R¹, -SO₂-R¹ or a biotinyl group; at the C-terminal end, Z is selected from the group consisting of OH, O R¹, NHR¹ or NR¹R²; R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N.

3. The pharmaceutical composition for use of claim 1 or the method of claim 2, wherein the subject is a human.

4. The composition for use of any one of claims 1 or 3 or the method of any one of claims 2 or 3, wherein the tetrapeptide is selected from the group consisting of SEQ ID No: 1, SEQ ID No: 2, SEQ ID No: 3, SEQ ID No: 4, SEQ ID No: 5, SEQ ID No: 6, SEQ ID No: 7, SEQ ID No: 8, SEQ ID No: 9, SEQ ID No: 10, SEQ ID No: 11, SEQ ID No: 12, SEQ ID No: 13, SEQ ID No: 14, SEQ ID No: 15, SEQ ID No: 16, SEQ ID No: 17, SEQ ID No: 18, SEQ ID No: 19, SEQ ID No: 20, SEQ ID No: 21, SEQ ID No: 22, SEQ ID No: 23, SEQ ID No: 24, SEQ ID No: 25, SEQ ID No: 26, SEQ ID No: 27, SEQ ID No: 28, SEQ ID No: 29 and SEQ ID No: 30.

5. The composition for use of claim 1 or claims 3-4, or the method of any one of claims 2-4, wherein the tetrapeptide is selected from the group consisting of SEQ ID No: 1, SEQ ID No: 12, SEQ ID No: 20 and SEQ ID No: 27.

6. The composition for use of any one of claims 1 or 3-5, or the method of any one of claims 2-5, wherein tetrapeptide is Pal-AKGD-OH, Pal-EKGD-OH, Pal-LKGD-OH, Pal-IRGD-OH.

7. The composition for use of any one of claims 1 or 3-6, or the method of any one of claims 2-6, wherein U of the tetrapeptide is independently selected from the group consisting of octanoyl (C8), decanoyl (C10), lauroyl (C12), myristoyl (C14), palmitoyl (C16), stearoyl (C18), biotinoyl, elaidoyl, oleoyle and lipoyle.

8. The composition for use of any one of claims 1 or 3-7, or the method of any one of claims 2-7, wherein U of the tetrapeptide is independently selected from the group consisting of lauroyl (C12), myristoyl (C14) and palmitoyl (C16).

9. The composition for use of any one of claims 1 or 3-8, or the method of any one of claims 2-8, wherein the tetrapeptide is present at from 0. 1ppm to 10,000ppm by weight of the composition.

10. The composition for use of any one of claims 1 or 3-9, or the method of any one of claims 2-9, further comprising additional further peptides selected from the group consisting of dipeptides, tripeptides, additional tetrapeptides, pentapeptides, hexapeptides and mixtures thereof.
